Europäisches Patentamt

(19) European Patent Office

Office européen des brevets

(11) Publication number: **0 241 715 B1**

(12) # EUROPEAN PATENT SPECIFICATION

(45) Date of publication of patent specification : **17.07.91 Bulletin 91/29**

(51) Int. Cl.$^5$: **G01N 3/10**

(21) Application number: **87103615.8**

(22) Date of filing : **12.03.87**

(54) Means and method for determining the tensile strength of ceramic material.

(30) Priority: **18.03.86 SE 8601262**

(43) Date of publication of application: **21.10.87 Bulletin 87/43**

(45) Publication of the grant of the patent: **17.07.91 Bulletin 91/29**

(84) Designated Contracting States: **DE FR GB IT**

(56) References cited: **US-A- 3 492 862** **US-A- 3 610 031**

(73) Proprietor: **ASEA CERAMA AB S-915 00 Robertsfors (SE)**

(72) Inventor: **Mattsson, Bertil Granbacken 3 S-915 00 Robertsfors (SE)** Inventor: **Nilsson, Jan Lövstigen 1 S-915 00 Robertsfors (SE)**

(74) Representative: **Boecker, Joachim, Dr.-Ing. Rathenauplatz 2-8 W-6000 Frankfurt a.M. 1 (DE)**

EP 0 241 715 B1

## Description

Apparatus and method for determining the tensile strength of ceramic material

The invention relates to an apparatus for determining the tensile strength of ceramic material or material having similar properties according to the precharacterising part of Claim 1. Such an apparatus, which is not specifically designed for determining the strength of ceramic material, is known from the US-A-3 610 031. The invention also relates to a method for determining the tensile strength of a ceramic material with the help of the afore-mentioned means.

It is extremely expensive to produce test rods of ceramic material, such as silicon nitride, or material having similar properties in the same form as for testing the tensile strength of metallic material having end portions which are larger in diameter than an intermediate portion. Furthermore, the poor ductility of ceramic material, and its brittleness means that flexural stress in the test rods has a far greater falsifying impact on the test results than when testing material of high elasticity which stretch considerably before breaking, as is the case with most metals. It is thus scarcely possible to use the usual types of machines for determining the tensile strength of ceramic materials.

From the US-A-3 610 031 an apparatus for determining the tensile strength of materials is known which exhibits a pressure chamber which is defined by a cylinder and two end portions with cylindrical holes which are provided with threads. The test body consists of a rod that has end portions of an increased diameter, these end portions being provided with threads. The two bodies to which the test rods secured by threading fit closely into the pressure chamber. The measurements are taken by a strain gage that is fitted to the test rod and is connected to measuring wires that are conducted through a bore in one of the two end portions, this end portion being permanently fixed to the pressure chamber.

US-PS-3 492 862 discloses a similar test apparatus as the afore-mentioned US-A-3 610 031. In this case, besides connecting the test rod to the end bodies by threads, a test body is suggested consisting of the proper test rod and end portion integral therewith which end portion are formed with several flanges spaced apart by circular slots which accommodating sealing means. In this test device also one of the end portions is secured to the pressure chamber.

The invention aims at developing an apparatus and a method for determining the tensile strength of ceramic material which enables testing the tensile strength of ceramic material at lower costs and with more reliable results than this is possible with the conventional means and methods.

To achieve this aim the invention suggests an apparatus for determining the tensile strength of ceramic material according to the introductory part of claim 1, which is characterized by the features of the characterizing part of claim 1.

A method for determining the tensile strength of a ceramic material is, according to the invention, characterized by the features of Claim 2.

Further developments of this method are characterized by the features of the additional claims 3 to 5.

The invention thus utilizes a hydraulic testing apparatus and a cylindrical test rod having the same cross section along its entire length. A test rod and two cylindrical bodies with bore holes fitting the test rod are joined together with an adhesive, preferably a cured glue, to form a unit. This unit is inserted into a cylindrical pressure chamber. Between the wall of the pressure chamber and the bodies seals are provided to produce a closed space which is defined by the wall of the pressure chamber and said two cylindrical bodies. This space communicates with a pressure medium source and pressure meter.

Tensile strength measuring is performed as follows : A test rod and two cylindrical bodies are joined together with adhesive. A suitable quantity of adhesive, preferably a cured glue such as ARALDIT$^R$, is inserted into the bore holes in the cylindrical bodies. The test rod is then inserted into the bodies and the glue allowed to set. Cured glue is preferably cured at an increased temperature, in the vicinity of 100°C, for instance. The unit thus formed is inserted into the pressure chamber, whereupon the space defined between the cylindrical bodies and the wall of the pressure chamber is filled with pressure medium, at gradually increasing pressure until the test rod fractures. The pressure at rupture is indicated.

The invention allows entirely cylindrical test rods to be used. A test rod produced from powder by hot isostatic pressing is suitably used, with the surface as obtained by the pressing. The unfinished surface after pressing has the same structure as the surface of a product. Since the surface structure of the test rod is the same as that of a product, the test result will be more reliable than in the case of using a test rod which has been machined, for instance by grinding. The cost of such test rods and of the testing amounts only to a fraction of the cost entailed when using test rods of conventional design. The results will be substantially more reliable than with the use of rods of conventional design and conventional machines for testing tensile strength.

The invention will now be described in greater detail with reference to the accompanying drawings showing

— by way of example — an embodiment of a means according to the invention.

The pressure chamber 1 comprises a cylinder 2 and two end plugs 3 and 4 screwed into the cylinder 2. An adjustment screw 5 is provided in the end plug 4. The cylinder 2 contains sealing rings 6a, 6b, which are supported by support rings 7, 8 with triangular cross-section, and support cylinders 10, 11 for transmitting axial forces exerted on the sealing rings to the end plugs 3, 4.

The two sealing rings 6a, 6b seal a space 15 which is defined by the wall 16 of the cylinder 2 and the bodies 13, 14. The space 15 communicates through an opening 17 in the wall of the cylinder 2, and a pipe 18, with a pressure medium source 20 supplying a pressure medium to the space 15. A pressure of 60 MPa (600 bar) is used in the example shown, but equipment for considerably greater pressure is feasible. A pressure meter 23 is connected to the space 15 via an opening 21 and a pipe 22.

The bodies 13, 14 are suitably made of steel. When the glue is cured at temperatures above room temperature, a strong grip is obtained on the test rod since steel has a higher coefficient of thermal expansion than ceramic. The adhesive force of the joint is thus increased. The pressure medium acts on the outer surface of the bodies and this, too, improves the grip. The test rod and end bodies are glued in a jig to ensure that they are completely parallel. To ensure high strength in the adhesive joint, the clearance between test rod 12 and the walls of the bore holes 24, 25 in the bodies 13 and 14 should be slight.

The unit 9 formed by test rod 12 and bodies 13 and 14 will be self-centering in the pressure chamber, so that no flexural forces arise thanks to the perfect geometrical shape. Deviations from a perfect geometrical shape would inevitably give rise of flexural stresses in the test rod. Any curvature or deviation from the central axis must be measured before testing, with the combined unit of the test rod and end bodies 13, 14 placed on two V-blocks at exactly the same distance from each other as that between the seals 6a, 6b in the pressure chamber 2, and with one resting point the same distance from the end surface of the body 13 as the distance between the sealing ring 6b and the end plug 3 in the cylinder 2. The position of the test rod in relation to the centre line is measured. The unit formed by test rod 12 and end bodies 13, 14, is then inserted into the pressure chamber 1 with the end body 13 in contact with the end plug 3 so that the seals 6a, 6b, abut at the prior resting points in the V-blocks. The screw 5 is used to check the position of the unit 9 in the pressure chamber before the testing begins.

The stress is nominally

$$\delta o = P \frac{A_1 - A_2}{A_2}$$

where P = the pressure in the space 15,
$A_1$ = the cross-sectional area of the end bodies,
$A_2$ = the cross-sectional area of the test rods.
If the point of fracture initiation can be localized, the fracture stress can be compensated

$$\frac{\Delta\delta}{\delta_o} = \frac{r \cdot e \cdot 16}{d^2}$$

where r = the distance from the centre line of the test rod to the fracture initiation point with positive sign towards the flexural centre,
e = half the centre deviation,
d = the diameter of the test rod.

The fracture stress is

$$\delta_t = \delta_o + \Delta\delta$$

When determining the tensile strength of a cylindrical test rod in the manner proposed according to the invention, the test rod used can have a surface as obtained by producing the test rod by hot isostatic pressing of powder (HIP-compression) and subsequent sand-blasting to remove the casing. The surface has not been affected by grinding and the test rod therefore has a surface corresponding to the surface of an industrial product, thus ensuring reliable and correct test values.

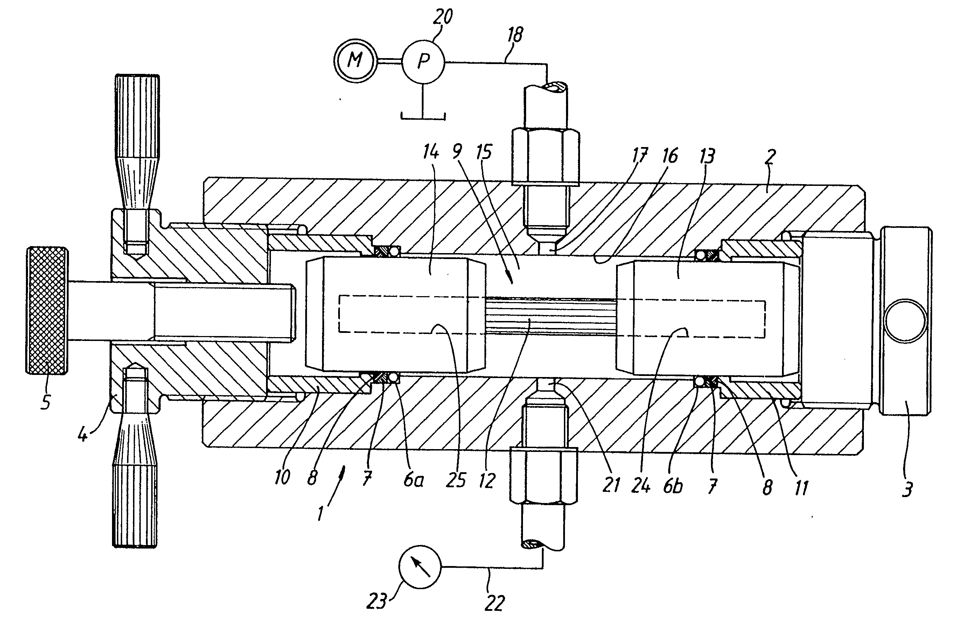

## Claims

1. Apparatus for determining the tensile strength of ceramic material or material having similar properties comprising a pressure chamber (1) having a cylindrical inner wall with two cylindrical bodies (13, 14) fitting therein, one at each end and sealing means between the circumferences of said bodies and said cylindrical inner wall, said bodies being provided with cylindrical bore holes (24, 25) for insertion and securing of respective ends of a test rod, whereby at least one of said bodies is axially displaceable in the pressure chamber (1), a pressure medium source (20) and a pressure meter (23) being connected to the space which is defined by said cylindrical inner wall of the pressure chamber (1), said sealing means, the surface of said test rod and the surface of said two bodies (13, 14), **characterized** in that the connection between respective ends of the test body and the two bodies (13, 14) is effected by means of adhesion, and in that the test body has the same diameter along its entire length.

2. Method for determining the tensile strength of ceramic material or material having similar properties wherein a test rod, formed of said material is inserted into a pressure chamber with a cylindrical inner wall in which the test rod is held between two cylindrical bodies (13, 14), positioned inside and at opposite ends of said chamber, which seal around their circumferences against the inner cylindrical wall of the pressure chamber, whereby at least one of said bodies is axially displaceable in said pressure chamber and whereafter a pressure medium is supplied to the space (15) formed between the wall of the pressure chamber (1) and said two bodies (13, 14), wherein test rod (12), which has the same diameter along its entire length, is inserted at its respective ends into bore holes (24, 25) in the two cylindrical bodies (13, 14) which bore holes fit the test rod, so that the test rod and the two cylindrical bodies are combined to form a unit with the aid of an adhesive between the test rod (12) and the bore holes (24, 25), and wherein the unit thus formed is placed in said pressure chamber (1), whereafter the pressure medium is admitted to the pressure chamber and the pressure at which the test rod fractures is recorded.

3. Method according to claim 2, **characterized** in that the test rod (12) and the cylindrical bodies (13, 14) are joined by means of a cured glue.

4. Method according to claim 3, **characterized** in that the glue is cured at a temperature between 50°C and 120°C.

5. Method according to claim 2 or 3, **characterized** in that the test rod is produced by hot isostatic pressing of powder, its surface remaining as obtained by the hot isostatic pressing process.

## Patentansprüche

1. Gerät zur Bestimmung der Zugfestigkeit von keramischem Material oder von Material mit ähnlichen Eigenschaften mit einer Druckkammer (1), welche eine zylindrische innere Wand hat, mit zwei in die Druckkammer passende zylindrische Körper (13, 14), von denen jeder an einem Ende angeordnet ist, und mit Dichtungsmitteln zwischen dem Umfang der genannten Körper und der genannten zylindrischen Innenwand, wobei die genannten Körper mit zylindrischen Bohrlöchern (24, 25) zur Einführung und Befestigung je eines Endes eines Teststabes versehen sind und mindestens einer der genannten Körper in axialer Richtung in der Druckkammer (1) verschiebbar ist, und mit einer Druckmittelquelle (20) und einem Druckmesser (23), die an den Raum angeschlossen sind, welcher von der genannten zylindrischen Innenwand der Druckkammer (1), den genannten Dichtungsmitteln, der Oberfläche des genannten Teststabes und der Oberfläche der genannten beiden Körper (13, 14) gebildet wird, **dadurch gekennzeichnet**, daß die Verbindung zwischen den entsprechenden Enden des Testkörpers und den beiden Körpern (13, 14) durch Kleben hergestellt ist und daß der Testkörper den gleichen Durchmesser längs seiner gesamten Länge hat.

2. Verfahren zur Bestimmung der Zugfestigkeit von keramischem Material oder von Material mit ähnlichen Eigenschaften, wobei ein aus dem genannten Material bestehender Teststab in eine Druckkammer mit einer zylindrischen Innenwand eingeführt wird, in welcher der Teststab zwischen zwei zylindrischen Körpern (13, 14) gehalten wird, die an gegenüberliegenden Enden im Inneren der genannten Kammer positioniert sind und längs ihres Umfanges gegen die innere zylindrische Wand der Druckkammer dichten, wobei mindestens einer der genannten Körper in axialer Richtung in der genannten Druckkammer verschiebbar ist, und worauf ein Druckmittel dem Raum (15) zugeführt wird, welcher von den Wänden der Druckkammer (1) und den genannten beiden Körpern (13, 14) begrenzt wird, und in welchem Raum der Teststab (12), der den gleichen Durchmesser längs seiner gesamten Länge hat, mit seinen entsprechenden Enden in Bohrlöcher (24, 25) in den beiden zylindrischen Körpern (13, 14) eingeführt wird, wobei die Bohrlöcher dem Teststab derart angepaßt sind, daß der Teststab und die beiden zylindrischen Körper mit Hilfe von Klebstoff zwischen dem Teststab (12) und den Bohrlöchern (24, 25) zu einer Einheit zusammengefügt werden, und wobei die so geformte Einheit in der Druckkammer (1) untergebracht wird, worauf das Druckmittel in die Druckkammer eingelassen wird und der Druck, bei welchem der Teststab zerreißt, registriert wird.

3. Verfahren nach Anspruch 2, **dadurch gekennzeichnet,** daß der Teststab (12) und die zylindrischen Körper (13, 14) mittels aushärtbarem Klebstoff miteinander verbunden werden.

4. Verfahren nach Anspruch 3, **dadurch gekennzeichnet,** daß der Klebstoff bei einer Temparatur zwischen 50°C und 120°C ausgehärtet wird.

5. Verfahren nach einem der Ansprüche 2 oder 3, **dadurch gekennzeichnet,** daß der Teststab durch isostatisches Heißpressen von Pulver hergestellt wird, wobei die Oberfläche so belassen wird, wie sie durch das isostatische Heißpressen gewonnen wird.

## Revendications

1. Dispositif pour la détermination de la résistance à la traction d'une céramique ou d'un matériau ayant des propriétés similaires, comprenant une chambre de pression (1) ayant une paroi intérieure cylindrique, avec deux blocs cylindriques (13, 14) ajustés à l'intérieur, un à chaque extrémité, et des moyens d'étanchéité entre les circonférences de ces blocs et la paroi intérieure cylindrique, ces blocs comportant des alésages cylindriques (24, 25) pour l'introduction et la fixation d'extrémités respectives d'un barreau de test, l'un au moins des blocs pouvant être déplacé axialement dans la chambre de pression (1), et une source de fluide sous pression (20), et un appareil de mesure de pression (23) étant reliés à l'espace qui est défini par la paroi intérieure cylindrique de la chambre de pression (1), les moyens d'étanchéité, la surface du barreau de test et la surface des deux blocs (13, 14), **caractérisé** en ce que l'accouplement entre des extrémités respectives du barreau de test et les deux blocs (13, 14) est effectué par adhérence, et en ce que le barreau de test a le même diamètre sur toute sa longueur.

2. Procédé pour déterminer la résistance à la traction d'un matériau consistant en une céramique ou d'un matériau ayant des propriétés similaires, dans lequel un barreau de test, formé par ce matériau, est introduit dans une chambre de pression ayant une paroi intérieure cylindrique, dans laquelle le barreau de test est maintenu entre deux blocs cylindriques (13, 14), positionnés à l'intérieur de la chambre et à des extrémités opposées de celle-ci, dont les circonférences sont en contact étanche avec la paroi cylindrique intérieure de la chambre de pression, l'un au moins de ces blocs pouvant être déplacé axialement dans la chambre de pression, et ensuite un fluide sous pression est introduit dans l'espace (15) qui est formé entre la paroi de la chambre de pression (1) et les deux blocs (13, 14), dans lequel le barreau de test (12), qui a le même diamètre sur toute sa longueur, est introduit au niveau de ses extrémités respectives dans des alésages (24, 25) qui sont formés dans les deux blocs cylindriques (13, 14), ces alésages s'ajustant exactement sur le barreau de test, de façon que le barreau de test et les deux blocs cylindriques soient combinés pour former un seul ensemble, à l'aide d'un adhésif intercalé entre le barreau de test (12) et les parois des alésages (24, 25), et dans lequel l'ensemble ainsi formé est placé dans la chambre de pression (1), après quoi le fluide sous pression est admis dans la chambre de pression et la pression à laquelle le barreau de test se rompt est enregistrée.

3. Procédé selon la revendication 1, **caractérisé** en ce que le barreau de test (12) et les blocs cylindriques (13, 14) sont réunis au moyen d'une colle polymérisable.

4. Procédé selon la revendication 3, **caractérisé** en ce que la colle est polymérisée à une température comprise entre 50°C et 120°C.

5. procédé selon la revendication 2 ou 3, **caractérisé** en ce que le barreau de test est fabriqué par compression de poudre, du type compression isostatique à chaud, et sa surface reste telle qu'elle est obtenue par l'opération de compression isostatique à chaud.